# EUROPEAN PATENT SPECIFICATION

(11) **EP 2 782 670 B1**
(45) Date of publication and mention of the grant of the patent: **06.09.2023**
(21) Application number: 11876333.3
(22) Date of filing: 21.11.2011
(51) Int. Cl.: B01J 37/03, B01J 37/18, B01J 23/80, B01J 21/04, B01J 21/06, B01J 23/72, B01J 35/00, B01J 35/10, C07C 31/08, C07C 67/40

(54) **COPER-ZICORNIA CATALYST AND METHOD OF USE AND MANUFACTURE**
KUPFER-ZIRKONIUM-KATALYSATOR SOWIE VERFAHREN ZU SEINER VERWENDUNG UND HERSTELLUNG
CATALYSEUR CUIVRE-ZIRCONE ET PROCÉDÉ DE FABRICATION ET D'UTILISATION

(43) Date of publication of application: 01.10.2014
(73) Proprietor: BASF Corporation, Florham Park, NJ 07932 (US)
(72) Inventor: MADON, Rostam, J., Flemington, NJ 08822 (US); NAGEL, Peter, Highlands, NJ 07732 (US); THAKUR, Deepak, S., Beachwood, OH 44122 (US)
(74) Representative: Maiwald GmbH
(86) International application number: PCT/US2011/061644
(87) International publication number: WO 2013/077835

(56) References cited:
- DE-A1- 2 753 634
- US-A- 4 480 122
- US-A- 5 196 561
- US-A- 5 892 102
- US-A1- 2003 060 655
- US-A1- 2004 242 917
- US-B2- 7 091 155
- WEN TAO ET AL: "CHARACTERIZATION OF CU/ZN/A1/ZR CATALYSTS FOR DIRECT SYNTHESIS OF ESTER FROM ALCOHOL", CUIHUA XUEBAO - JOURNAL OF CATALYSIS, BEIJING, CN, vol. 19, no. 1, 1 January 1998 (1998-01-01), pages 77-80, XP002928852, ISSN: 0253-9837
- HIROYUKI KAMATA ET AL: "Steam Reforming of Dimethyl Ether over Cu/ZnO/ZrO2 and .GAMMA.-Al2O3 Mixed Catalyst Prepared by Extrusion", JOURNAL OF THE JAPAN PETROLEUM INSTITUTE, vol. 51, no. 3, 1 January 2008 (2008-01-01), pages 157-164, XP055218220, ISSN: 1346-8804, DOI: 10.1627/jpi.51.157

## Description

### TECHNICAL FIELD

The present invention relates to catalysts, methods for preparing catalysts and methods using the catalyst to produce esters, for example, for converting ethanol to ethyl acetate.

### BACKGROUND

Dehydrogenation is a chemical reaction that involves the elimination of hydrogen (H₂) and is used in large scale industrial processes or smaller scale laboratory procedures. Copper is a known catalyst for dehydrogenation reactions, however, the activity and selectivity of the catalyst can be significantly enhanced by the correct use of promoters as well as the correct method of preparation of the catalyst. Zinc oxide (ZnO), zirconium oxide (ZrO₂) and aluminum oxide (Al₂O₃) have been used as components with various Cu-containing catalysts. In general, such Cu-containing catalysts are made using ZnO, ZrO₂ and Al₂O₃ precursors, such as soluble salts of the components such as copper nitrate, zinc nitrate, zirconyl nitrate, and aluminum nitrate and their simultaneous precipitation with a base such as sodium carbonate or bicarbonate.

Dehydrogenation can be used in reactions with alcohols. For example, dehydrogenation may be used to dehydrogenate methanol to give formaldehyde, ethanol to give acetaldehyde, 1-propanol to give propionaldehyde, isopropanol to give acetone, I-butanol to give butyraldehyde, 2-butanol to give methyl ethyl ketone, isobutanol to give isobutyraldehyde, and also for dehydrogenating the isomeric primary and secondary pentanols, hexanols, heptanols, octanols, nonanols, decanols, undecanols and dodecanols to give the corresponding aldehydes and ketones respectively.

One such product formed from the dehydrogenation of ethanol includes ethyl acetate, which is commonly used as a solvent. The synthesis of ethyl acetate typically utilizes reactions between ethanol and acetic acid or the dehydrogenation of ethanol. Recently, interest in synthesizing ethyl acetate by dehydrogenating ethanol has increased because surplus ethanol feedstock can be used. Examples of catalysts and methods known in the art for forming ethyl acetate include the catalyst described in U.S. Patent No. 7,091,155 B2 and the catalysts and methods disclosed in Chinese patent ZL Patent No. 92100590.3 US2004/242917 discloses a method for making ethyl acetate via the dehydrogenation of ethanol using a Cu-Zn-Al-Zr catalyst. The catalyst may be prepared by coprecipitation using a hydroxide from a solution of the corresponding nitrates.

It would be desirable to provide dehydrogenation catalysts, methods for their manufacture and methods of use which exhibit higher catalytic activity than existing catalysts.

### SUMMARY

A first aspect of the present invention pertains to a catalyst as defined in claim 5, comprising CuO, ZnO, Z₁O₂ and Al₂O₃, wherein the Al₂O₃ in the catalyst is derived from a highly dispersible alumina, instead of aluminum nitrate. As used herein, the term "dispersible alumina" refers to the amount of alumina that becomes colloidal at a certain pH, which is typically in the acid range, a process that is referred to as acid peptizing, Acid peptizing results in the formation of particles that are less than 1 micron (µm). A dispersible alumina has 50% or greater dispersibility in water after peptizing at a pH of 2 to 5. Other examples of alumina 60% or greater dispersibility, 70% or greater dispersibility, 80% or greater dispersibility, or 90% or greater dispersibility in water after peptizing at a pH of 2 to 5 are included in this definition of dispersible alumina. As used herein, the percent dispersibility of alumina refers to the percentage of alumina that is less than 1 micron in size in the acidic solution after peptizing at a pH from about 2 to about 5. Non-limiting examples of aluminas that are dispersible include boehmite or pseudo-boehmite aluminas,

The dehydrogenation catalyst of one or more embodiments may exhibit an XRD pattern containing boehmitic peaks at 2 theta values of about 14.2° and 28.1°.

In one variant, the dispersible alumina may have a dispersibility of at least 70% or greater or 90% or greater. The dispersible alumina may be selected from boehmite, pseudoboehmite, and mixtures thereof. In one or more embodiments, at least a portion of the dispersible alumina may be replaced with nondispersible alumina. For example, up to 99% by weight of the dispersible alumina may be replaced with The non-dispersible alumina may be selected from γ-alumina, η-alumina, χ-alumina, other transitional aluminas, boehmite, pseudoboehmite, gibbsite, bayerite, and mixtures thereof.

A second aspect of the present invention pertains to a process of preparing the dehydrogenation catalysts disclosed herein as defined in claim 1. In one or more embodiments, the process of preparing the dehydrogenation catalysts includes peptizing a highly dispersible alumina to form a peptized alumina and reacting the peptized alumina with precursors of ZrO₂, ZnO and CuO.

The process includes forming the peptized alumina by forming a slurry by peptizing a dispersible alumina in an acid at a pH between 2 and 5 and a temperature of about 20°C to 30 °C. In a specific embodiment, the process may include peptizing a dispersible alumina at a pH of about 3 and a temperature of about 25 °C. The dispersible alumina of one or more embodiments may be replaced with non-dispersible alumina. In a specific variant, up to 99% by weight of the dispersible alumina may be replaced with nondispersible alumina.

The process includes forming a ZrO₂ precursor by forming a slurry of zirconyl nitrate and water in a separate vessel from the peptized alumina. The slurry of zirconyl nitrate is then mixed with the slurry formed above by peptizing a dispersible alumina to form a mixed slurry or a new slurry. The slurry of zirconyl nitrate and water is formed at a pH of less than about 1.5 and a temperature in the range of about 20 °C to 30 °C. In one variant, the slurry of zirconyl nitrate and water may be formed at a pH of about 1.0 and a temperature of about 25°C. After mixing the two slurries to form a mixed slurry, the process may also include maintaining the pH of the mixed slurry at less than about 1.5 and a temperature in the range of about of about 20 °C to 30°C. In a specific embodiment, the process includes maintaining the mixed slurry at a pH of about 1.0 at a temperature of about 25 °C. As used herein, the mixed slurry may be referred to as a new slurry or a first reaction product.

The process includes forming a Cu and Zn precursor by forming a solution of copper nitrate and zinc nitrate and mixing the solution with the mixed slurry formed above. As used herein, the copper nitrate and zinc nitrate solution may be referred to as a second reaction product. The copper nitrate and zinc nitrate solution is formed at a pH of less than about 1.5 and at a temperature in the range of about 30 °C to 50 °C. In one variant, the process includes forming a solution of copper nitrate and zinc nitrate at a pH of about 1.0 at a temperature of about 40°C. The process includes mixing the copper nitrate and zinc nitrate solution (second reaction product) with the mixed slurry (or first reaction product). During mixing, the process may include maintaining the pH at less than about 1.5, while raising the temperature to a range of about 30 °C to 50 °C to create an acidic slurry containing copper nitrate, zinc nitrate, zirconyl nitrate, and alumina. In one variant, the step of mixing the copper nitrate and zinc nitrate solution with the mixed slurry includes maintaining the pH at about 1.0, while the temperature is raised or increased to about 40 °C,

The process includes adding a basic solution and the acidic slurry formed above to a vessel containing a heel of water. The process includes forming a basic solution of sodium carbonate or sodium bicarbonate at a temperature in the range of about 30 °C to 50 °C in one vessel and forming a heel of water in a separate vessel at a temperature in the range of about 30 °C to 50 °C. The solution of sodium carbonate or sodium bicarbonate and/or the heel of water may be formed at a temperature of about 40 °C. The process includes adding the basic solution and the acidic slurry prepared above to the vessel containing the heel of water so that a precipitation reaction occurs at a pH of about 6 to 7 and at a temperature in the range of about 30 °C to 50 °C to provide a precipitate slurry. The acidic slurry and the basic solution may be added to the heel of water so that the precipitation reaction may occur at a pH of about 6.5 at a temperature of about 40 °C.

The process may also include aging the precipitate slurry formed from the precipitation reaction for a time in the range of about 15 minutes to 15 hours at a temperature in the range of about 30 °C to 70 °C. In one variant, the precipitate slurry may be aged for about 2 hours at a temperature of about 60 °C. The precipitate slurry may be filtered and washed to provide or form a filter cake. The filter cake may be dried to form a dry filter cake or powder and calcined to decompose carbonates to oxides, in accordance with one or more embodiments of the process.

A third aspect of the present invention pertains to a method as defined in claim 9 for dehydrogenating alcohol, which may include ethanol. In one or more embodiments, the method includes contacting an alcohol-containing stream with a dehydrogenation catalyst as described herein and converting the alcohol to ester, which may comprise ethyl acetate. Prior to contacting the alcohol-containing stream, the method includes reducing the dehydrogenation catalyst in a stream containing hydrogen.

### BRIEF DESCRIPTION OF THE DRAWINGS

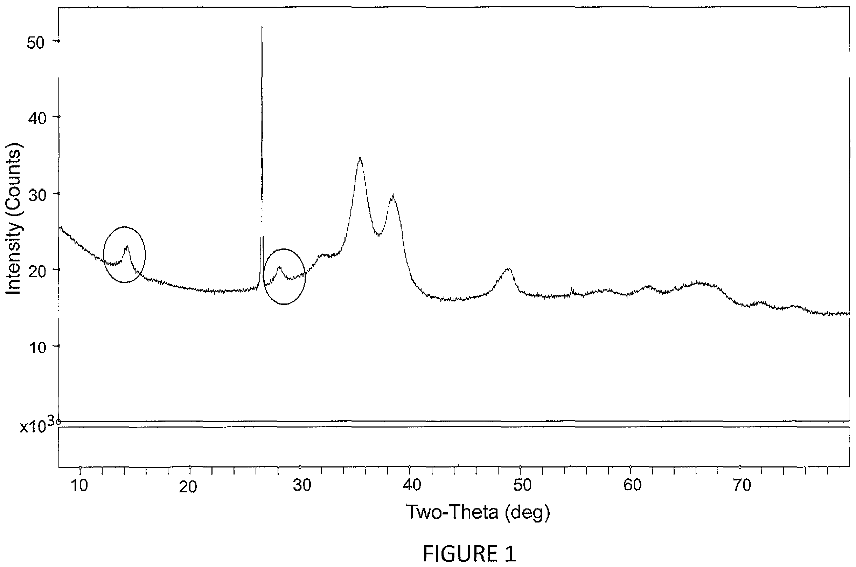

Figure 1 is an XRD pattern of the copper-zirconia catalyst prepared according to Example 1.

### DETAILED DESCRIPTION

Before describing several exemplary embodiments of the invention, it is to be understood that the invention is not limited to the details of construction or process steps set forth in the following description. The invention is capable of other embodiments and of being practiced or being carried out in various ways.

A first aspect of the present invention pertains to a dehydrogenation catalyst comprising Cu, ZnO, ZrO₂ and Al₂O₃. In one embodiment, the catalyst exhibits increased dehydrogenation activity compared to conventional catalysts used in dehydrogenation reactions. In one or more embodiments, the catalyst comprises Cu, ZnO, ZrO₂ and Al₂O₃ and the catalyst can be prepared by using highly dispersible alumina. In specific embodiments, the Cu of the catalyst is highly dispersed by reaction of precursors of ZrO₂, ZnO and CuO and an alumina formed by peptizing of boehmite or pseudoboehmite. The dehydrogenation catalyst comprises Cu, ZnO, ZrO₂ and Al₂O₃, wherein the CuO is present in an amount of about 10% to about 75% by weight of the catalyst. The catalyst may be prepared by reaction of an alumina formed by peptizing of boehmite or pseudoboehmite and precursors of ZrO₂, ZnO and CuO, wherein the alumina has a dispersibility of at least about 50%. In other specific embodiments, the alumina may have a dispersibility of at least about 70% and at least about 90%.

In one or more specific embodiments, the Cu has a surface area exceeding 15 m²/g of the catalyst. In an even more specific embodiment, the Cu has a surface area exceeding 20 m²/g of the catalyst. The catalyst of one or more embodiments may include Cu with a surface area exceeding 25 m²/g of the catalyst. In one or more embodiment, CuO is present in the catalyst in an amount in the range of from about 30% to about 70% by weight of the catalyst. In a specific embodiment, CuO is present in an amount in the range from about 40% by weight to about 60% by weight. In a more specific embodiment, CuO is present in an amount in the range from about 45% to about 55% by weight. In one or more embodiments, ZnO, ZrO₂ and Al₂O₃ comprise the remaining amount of the catalyst, or more specifically, from the range of about 70% to about 30% by weight of the catalyst, and more specifically in the range of about 60% to about 40% by weight of the catalyst. ZnO is present in an amount in the range from about 5 weight % to about 50 weight %, ZrO₂ is present in an amount in the range from about 1 weight % to about 30 weight % and Al₂O₃ is present in an amount in the range from about 5 weight % to about 40 weight %.

The catalyst according to one or more embodiments may have a BET total surface area of greater than about 140 m²/g. The catalyst may exhibit an XRD pattern containing boehmitic peaks at 2 theta values of about 14.2° and 28.1°.

The alumina utilized in the catalysts described herein is characterized as peptized until the desired dispersibility or a "dispersible alumina," as defined above, is achieved. According to one or more specific embodiments, the use of a highly dispersible alumina allows more of the surface area of the Cu to be exposed for reaction and thus provides a higher catalytic activity. In one or more embodiments, the peptized alumina has a particle size of 1 µm or less. The alumina has 50% or greater dispersibility in water after peptizing at a pH of 2 to 5. In other words, the percentage of alumina having a particle size of 1 µm or less in water after peptizing at a pH of 2 to 5 is at least 50%. In a more specific embodiment, the alumina has 80% or greater dispersibility in water after peptizing at a pH of 2 to 5. Other suitable alumina may have 90% or greater dispersibility in water after peptizing at a pH of 2 to 5. In one or more alternative embodiments, non-dispersible alumina may be used in combination with dispersible alumina. In such embodiments, the non-dispersible alumina is milled into a fine powder before use.

The catalyst includes Al₂O₃ which may be formed or derived from boehmite, pseudoboehmite and combinations thereof. Suitable boehmite and pseudoboelimites have 70% or greater dispersibility in water after peptizing at a pH of 2 to 5. For example, suitable aluminas are available from Sasol North America Inc. of Houston, Texas, under the trademarks Catapal^{®}, Pural^{®}, Dispersal^{®}, and Dispal^{®}. Examples of aluminas that may be utilized in the catalysts described herein include aluminas available under the trade names Catapal A, B, Cl, and D and Pural SB. A specific example of a suitable alumina is available under the trade name CATAPAL D and has a particle size d₅₀ of about 40 µm. The alumina available under the trade name CATAPAL D also has a BET surface area of 220 m²/g and a pore volume of about 0.55 ml/g after activation at 550 °C for 3 hours.

As will be understood, other sources of alumina can be used and include such diverse materials as aluminum nitrate. Some dispersible alumina sources are thought to be unsuitable for industrial scale applications because of their tendency to gel or become solid under normal operating conditions in an industrial or large-scale setting. Accordingly, many known catalysts and methods of making and using such catalysts utilized aluminum nitrate as an alumina source. Modifying these known alumina sources, for example, aluminum nitrate and aluminum powders, were considered, however, none of these yielded the high Cu dispersion and enhanced catalytic activity desired. The dispersible alumina of the present invention is selected, despite the many issues regarding its use, and modified by peptizing to achieve the high Cu dispersion. Specifically, as stated above, the Cu of one or more catalysts described herein is highly dispersed by the reaction of an alumina derived by peptizing of boehmite or pseudoboehmite and precursors of ZrO₂, ZnO and CuO.

In one or more embodiments, at least a portion of the dispersible alumina in the catalyst may be replaced with nondispersible alumina. Suitable nondispersible alumina include γ-alumina, η-alumina, χ-alumina, other transitional aluminas, boehmite, pseudoboehmite, gibbsite, bayerite, and mixtures thereof.

The catalyst according to one or more embodiments includes ZrO₂, ZnO and CuO which are formed from various precursors. The ZrO₂ precursor is zirconyl nitrate. When zirconyl nitrate is used as the zirconia precursor, the ZrO₂ precursor is provided by forming a slurry of zirconyl nitrate and water. In such embodiments, the reaction mixture or the zirconyl nitrate and water slurry is maintained at a pH of less than 1.5 or 1. In one or more specific embodiments, the reaction mixture or the zirconyl nitrate slurry is maintained at a pH of about 1. The reaction mixture is maintained or has a temperature in the range from about 20 °C to about 30 °C. In one or more embodiments, the temperature of the zirconyl nitrate slurry may be maintained at a temperature of about 25°C.

The CuO precursor is copper nitrate. The ZnO precursor is zinc nitrate. The catalyst is formed by first reacting a ZrO₂ precursor with the peptized alumina to provide a first reaction product, a mixed slurry or a new slurry. A second reaction is then performed in which the CuO and ZnO precursors are reacted in a separate vessel to form or provide a second reaction product. The first reaction product and second reaction product are then subsequently mixed together.

A second aspect of the present invention pertains to a method of preparing a catalyst as described herein. In one or more embodiments, the method includes peptizing the highly dispersible alumina to form a peptized alumina and reacting the peptized alumina with precursors of ZrO₂, ZnO and CuO, as described above,

A highly dispersible alumina, as otherwise described herein, is prepared by adding the alumina to water to provide approximately 5 wt% to 35 wt% solids. The alumina and water mixture is mixed at high shear for approximately one hour to form a slurry. The alumina and water mixture is maintained at a pH in the range from about 2 to about 5 during the mixing process at a temperature in the range from about 20 °C to about 30 °C. In a specific embodiment, the alumina and water is maintained at a pH of about 3 during the mixing process. In an even more specific embodiment, the temperature of the alumina and water is maintained at about 25 °C. The pH of the alumina and water mixture is maintained by adding an amount of acid to the mixture. Examples of suitable acids include nitric acid, formic acid, other known acids and combinations thereof. As described herein, in one or more embodiments the dispersible alumina may be replaced with nondispersible alumina. For example, up to 99% of the dispersible alumina may be replaced with nondispersible alumina that may include γ-alumina, η-alumina, χ-alumina, other transitional aluminas, boehmite, pseudoboehmite, gibbsite, bayerite, and mixtures thereof.

Prior to a first reaction of the peptized alumina and the ZrO₂ precursor, the ZrO₂ precursor is prepared as a slurry in a separate vessel from the highly dispersible alumina. The process includes maintaining the ZrO₂ precursor a low pH and a controlled temperature. The ZrO₂ precursor is maintained at a pH of less than about 1.5. In one or more specific embodiments, the ZrO₂ precursor is maintained at a pH in the range from about 1.0 to about 1.5. In a more specific embodiment, the ZrO₂ precursor is maintained at a pH of about 1.0. The temperature of the ZrO₂ precursor of one or more embodiments is maintained at a temperature in the range from about 20° C to about 30°C prior to the first reaction with the peptized alumina. In one or more specific embodiments, the ZrO₂ precursor is maintained at a temperature in the range from about 22 °C to about 28 °C, or, more specifically, in the range from about 24 °C to about 26 °C. In one variant, the ZrO₂ precursor is maintained at a temperature of about 25°C.

The dispersed alumina and water slurry is added to the slurry of the ZrO₂ precursor and the alumina slurry and ZrO₂ precursor are well mixed for a duration from about 30 minutes to about 60 minutes to form a first reaction product, new slurry or mixed slurry. While the alumina slurry and the ZrO₂ precursor are mixed, the pH is maintained at less than about 1.5, In one or more embodiments, while the mixed slurry or first reaction product is formed, the process includes maintaining the pH at about 1 or as close to 1 as possible. The temperature is also maintained at a range from about 20 °C and about 30°C or, more specifically, at 25 °C.

In one or more embodiments, the CuO and ZnO precursors are prepared separately for reaction with the first reaction product, new slurry or mixed slurry. In a separate vessel, a solution of the CuO precursor and ZnO precursor is prepared to form a second reaction product. The second reaction product is provided by forming a solution of copper nitrate and zinc nitrate in a separate vessel. The temperature of second reaction product is maintained at a temperature in the range from about 30°C to about 50 °C. In one or more specific embodiments the temperature of the second reaction product is maintained at about 40 The pH of the second reaction product is maintained at a pH of less than about 1.5 or, in a more specific variant, at about 1. In one or more specific embodiments, the second reaction product is maintained at this pH by the addition of soda ash, or other suitable sodium source, for example, sodium hydroxide, sodium carbonate or sodium bicarbonate.

The second reaction product is then added to the first reaction product or mixed slurry. The first reaction product and the second reaction product are well mixed for a duration from about 30 minutes to about 60 minutes. The temperature and/or pH may be adjusted or controlled to create an acidic slurry. The acidic slurry may containing copper nitrate, zinc nitrate, zirconyl nitrate and alumina.

The first reaction product and the second reaction product are maintained at a pH of less than about 1.5, or in a more specific embodiment, at about 1 or as close to about 1 as possible. The temperature is controlled. The temperature of the first reaction product and the second reaction product is raised and maintained at a temperature in the range from about 30 °C to about 50 °C. In one or more specific embodiments, the temperature of the first reaction product and the second reaction product is raised and maintained at a temperature of about 40°C

The acidic slurry formed from the first reaction product and the second reaction product is then combined with a precipitation solution and a heel of water to form a precipitate slurry. The precipitation solution includes a basic solution of one or more of sodium carbonate and sodium bicarbonate and is formed separately from the heel of water. The precipitation solution is formed at a temperature and/or has a temperature in the range from about 30 °C to about 50 °C or, in one or more specific embodiments, a temperature of about 40 °C. In one or more embodiments, the slurry is formed by adding the acidic slurry and the precipitate solution simultaneously and slowly to a separate vessel containing a heel of water to form a precipitate slurry. The heel of water has a temperature in the range from about 30 °C to about 50 °C or, in one or more specific embodiments, a temperature of about 40 °C. This simultaneous addition of the acidic slurry and the precipitation solution improves the consistency in the precipitation of the carbonates.

After addition, the first and second reaction products and the precipitation solution are well stirred for a duration of about 90 minutes. In one or more embodiments, precipitation reaction is performed or carried out at a pH that is controlled, for example, by adjusting the flow of the first and second reaction product and/or the flow of the precipitation solution. The pH is controlled to an amount in the range from about 6 to about 7 or, more specifically, in the range from about 6.5 to about 6.7. In one or more specific embodiments, the pH is controlled at about 6.5. The precipitation is carried out at a temperature in the range from about 30 °C to about 50 °C, or more specifically, a temperature of about 40 °C.

In one or more embodiments, the precipitate slurry is digested or aged for a duration of about 15 minutes to about 15 hours. In a specific embodiment, the precipitate slurry is digested or aged for a duration of about 1 hour to about 3 hours. In an even more specific embodiment, the precipitate slurry is digested or aged for a duration of about 2 hours. The temperature of the precipitate slurry is increased to a temperature in the range from about 30 °C to about 70 °C during aging or, more specifically, to a temperature of about 60 °C. In one variant, the pH of the precipitate slurry during digestion or aging is not controlled. In such embodiments, the pH of the precipitate slurry undergoes some changes by cyclically increasing and decreasing, though the amount of increase and decrease may not be uniform. During the digestion or aging process, the color of the slurry changes from blue to green. In one variant, the method includes filtering and washing the slurry to form a filter cake. The method may also include drying the filter cake to form a dry filter cake or dried powder. The dry filter cake or dried powder may then be calcined to decompose any carbonates to oxides. In one or more embodiments, the dry filter cake or dried powder may be calcined for a duration of about 2 hours at a temperature of about 350 °C.

In one or more embodiments, the resulting catalyst, before reduction of the copper oxide to form copper metal, includes cupric oxide in an amount in the range from about 10% by weight to about 75% by weight. In one variant, ZnO was present in the resulting catalyst, before reduction, in an amount in the range from about 5% by weight to about 70% by weight. In one or more embodiments, the catalyst includes ZrO₂ in an amount in the range from about 1% by weight to about 50% by weight, before reduction. In one or more embodiments, the catalyst includes alumina in an amount in the range from about 5% by weight to about 70% by weight, before reduction.

In one or embodiments, the prepared catalyst is further reduced. A variant of the reducing step utilizes a hydrogen-containing gas. Specifically, such methods may include heating the catalyst to a temperature in the range from about 150°C to about 200 °C while flowing nitrogen gas at atmospheric pressure over the catalyst in a reactor. In one or more specific embodiments, the catalyst is heated to a temperature in the range from about 165 °C to about 185 °C in flowing N₂. In a more specific embodiment, the catalyst is heated to a temperature of about 170 °C in flowing N₂. The nitrogen is replaced incrementally by hydrogen. The temperature may be slowly and incrementally increased to a maximum of about 220 °C.

The resulting catalyst includes copper metal, formed form the reduction of the CuO precursor. The catalyst also includes ZrO₂, which functions as a chemical promoter, while ZnO and alumina function as structural promoters. In one or more embodiments, at least the ZnO and ZrO₂ are closely associated with the copper metal.

A third aspect of the present invention pertains to a method of converting alcohol to an ester or otherwise dehydrogenating an alcohol. One or more embodiments of the method include contacting an alcohol-containing fluid stream with a catalyst as described herein and dehydrogenating the alcohol to an ester. The alcohol-containing fluid stream may be flowed at 1h⁻¹ LHSV with a hydrogen-containing gas that is flowed at 4.2 h⁻¹ GHSV. The catalyst utilized in the method to convert alcohol is reduced in a stream containing hydrogen. In one or more embodiments, the alcohol may include ethanol and the ester that is formed may include ethyl acetate.

Without intending to limit the invention in any manner, embodiments of the present invention will be more fully described by the following examples.

### EXAMPLES:

Two catalysts were prepared and the catalytic activity of each catalyst was measured. Both catalysts, Example 1 and Comparative Example 2, included CuO, ZnO, ZrO₂ and Al₂O₃. Example 1 was made with highly dispersible alumina having a dispersibility greater than about 90%, available under the trade name Catapal D from Sasol North America, Inc. Comparative Example 2 was made with aluminum nitrate. The components and quantities of the components used to make the final catalyst of Example 1 and Comparative Example 2 catalyst in the oxide or unreduced form are provided in Table 1.

**Table 1**

| **Example 1** | | | | **Comparative Example 2** | | |
|---|---|---|---|---|---|---|
| | | Amount, g | | | | Amount, g |
| **Reagents (solutions)** | | | | **Reagents (solutions)** | | |
| | 16% Copper in Nitrate solution | 1919.6 | | | 16% Copper in Nitrate solution | 1919.6 |
| | 16.5% Zinc in Nitrate solution | 579.4 | | | 17% Zinc in Nitrate solution | 562.4 |
| | Al Nitrate solution | N/A | | | 7.2% Al in Nitrate solution | 927.4 |
| | 14.6% Zirconia in Nitrate | 510.2 | | | 26.7% Zirconia in Nitrate | 262.7 |
| | Al₂O₃ (@ 19% VF solids) | 663.2 | | | Al₂O₃ (@19% VF solids) | N/A |
| | Water to be added for dilution | 2841.3 | | | Water to be added for dilution | 2994.1 |
| Sodium Carbonate | | 1146.7 | | Sodium Carbonate | | 1146.7 |
| Water | | 3631.1 | | Water | | 3631.1 |
| Total carbonate solution | | 4777.8 | | Total carbonate solution | | 4777.8 |
| Water Heel | | 2124.6 | | Water Heel | | 2124.6 |
| Total wt of solution | | 4461.6 | | Total wt of solution | | 3672.1 |
| Water in mix | | 1853.0 | | Water in mix | | 1700.2 |
| % total metal concentration in solution | | 25.3 | | % total metal concentration in solution | | 27.6 |
| Water for required concentration | | 4694.3 | | Water for required concentration | | 4694.3 |
| **Grams of metal in final catalyst** | | | | **Grams of metal in final catalyst** | | |
| | Cu | 307.1 | | | Cu | 307.1 |
| | Zn | 95.6 | | | Zn | 95.6 |
| | Al from nitrate | N/A | | | Al from nitrate | 66.7 |
| | Al from solid alumina | 66.7 | | | Al from solid alumina | N/A |
| | Zr | 74.3 | | | Zr | 52.0 |
| | Total g of metal | 543.8 | | | Total g of metal | 521.4 |

Example 1 is prepared according to the methods of preparing a catalyst composition described herein. As Comparative Example 2 utilized aluminum nitrate as an alumina source and thus, did not contain any solid alumina. Accordingly, a different preparation procedure was utilized to form Comparative Example 2. Comparative Example 2 was formed using copper nitrate, zinc nitrate, zirconyl nitrate, and aluminum nitrate precursors, which were all mixed together without the need for the initial interaction of zirconyl nitrate with aluminum nitrate. The mixture had a pH of 2.5. A slurry was formed by precipitating the mixture with soda ash at a pH of 7 and temperature of 60 °C, The slurry was then digested at a temperature of about 60 °C for a duration of about 90 minutes. The filtration, washing, drying, and calcination steps for preparing Comparative Example 2 were the same as for Example 1. Example 1 and Comparative Example 2 were reduced at approximately 210°C using a gas containing 5% hydrogen in nitrogen, as described herein, Table 2 provides the analyses of Example 1 and Comparative Example 2 before and after reduction.

**Table 2: Analyses of Example 1 and Comparative Example 2**

| **Example** | **Example 1** | **Comparative Example 2** |
|---|---|---|
| **Analyses** | | |
| %CuO | 48.0 | 51.0 |
| %ZnO | 16.0 | 15.5 |
| % ZrO2 | 12.1 | 11.5 |
| %A1203 | 23.9 | 22.0 |
| %Na2O | 0.02 | 0.04 |
| **After reduction** | | |
| % Cu | 42.5 | 45.5 |
| % rest of components as oxides | 57.5 | 54.5 |
| BET total surface area, m²/g | 142 | 134 |

The Cu surface areas of reduced Example 1 and reduced Comparative Example 2 as prepared in Example 1 were measured by a standard procedure described by G.C. Chinchen et al. in Journal of Catalysis (1987), vol 103, pages 79 to 86. After reducing, a reduced metallic Cu surface is obtained on both Example 1 and Comparative Example 2. A gas containing 2 wt% N₂O in helium at a temperature of 60°C is flow through reduced Example 1 and Comparative Example 2 for 10 minutes. It is believed that the nitrous oxide decomposes on the copper surface of the catalysts and the resulting N₂ evolved is measured via a thermal conductivity detector, while the oxygen atoms remain attached to the copper. Each oxygen atom is attached to two surface Cu atoms. The amount of nitrogen evolved gives a measure of the number of number of oxygen atoms, and thus copper atoms available on the surface of the catalyst. The surface area of a Cu atom is 6.8 ×10⁻¹⁶ cm²/atom, By multiplying the number of Cu atoms by the area of each atom the copper surface area of the catalyst is derived. The Cu dispersion, which is defined as surface copper atoms as a percentage of all copper atoms, and Cu surface areas of Example 1 and Comparative Example 2 are shown in Table 3.

**Table 3: Cu dispersion and Cu surface area**

| | **% Cu dispersion** | **Cu surface area, m²/g of catalyst** |
|---|---|---|
| **Example 1** | 10.4 | 28.4 |
| **Comparative Example 2** | 3.0 | 8.8 |

As is evident from Table 3, Example 1 made with dispersible alumina and with the method described above exhibits a significant increase in the Cu dispersion and surface area compared to Comparative Example 2, which was made with aluminum nitrate instead of a dispersible alumina.

A sample made in accordance with Example 1 was submitted for X-ray diffraction analysis using standard techniques. The sample was ground in a mortar and pestle. The resultant powder was then backpacked into a flat plate mount for use in reflection mode. X-ray diffraction was performed on a θ-θ PANalytical X'Pert Pro MPD X-ray diffractometer with Cu_{kα} radiation. The generator settings were voltage 45kV and current 40mA. The diffractometer optics utilized Bragg-Brentano geometry, a ¼° divergence slit, 0.04 radian soller slits, 15mm beam mask and ½° anti-scatter slit. The data collection range was 8° to 80° two theta (2θ) using a step size of 0.0334° and counting for 240 seconds per step. As illustrated in Figure 1, the catalyst exhibits an XRD pattern containing boehmitic peaks at 2 theta values of about 14.2° and 28.1°.

As an example of the dehydrogenation catalysis of the catalysts described herein, ethanol was reacted with Catalysts 1 and Comparative Example 2, to form ethyl acetate. Before use Example 1 and Comparative Example 2 were reduced in a hydrogen containing gas to obtain the final catalyst comprising of Cu, ZnO, ZrO₂, and Al₂O₃, as described herein. The catalyst reduction procedure utilized includes heating Example 1 and Comparative Example 2 to a temperature of about 170°C in 250 sccm flowing nitrogen gas at atmospheric pressure. Subsequently hydrogen is introduced to the nitrogen flow in a step-wise fashion for up to about 1 hour for each step, starting at low concentration as outlined below:
1.12 cc/min H₂ in 238 cc/min N₂
2. 25 cc/min H₂ in 225 cc/min N₂
3. 50 cc/min H₂ in 200 cc/min N₂
4. 125 cc/min H₂ in 125 cc/min N₂
5. 200 cc/min H₂

Once reduction was completed, the temperature was increased to 220°C in 200 sccm hydrogen, and then reactor was pressurized to 10 bars absolute. Once temperature and pressure was stable, the ethanol feed stream was introduced to the reactor at 1 h⁻¹ LHSV (23.7 g/li) while maintaining hydrogen flow at 4.2 h⁻¹ GHSV (2 seem).

To measure the catalytic activity of Example 1 and Comparative Example 2, a stainless steel reactor tube having dimensions of approximately 84 cm in length, 2.5 cm outer diameter and 2.1 cm inner diameter was utilized. The reactor tube was equipped with a thermocouple well having an outer diameter of 0.47 cm. The thermocouple well ran through the center of the tube and housed 5 thermocouples. The thermocouples were spaced evenly from the top to the bottom of the catalyst bed. The average temperature of the thermocouples was used to control temperature. Thirty cc of each of Example 1 and Comparative Example 2 having a particle size in the range from about 30 mesh to about 50 mesh was mixed with 30 cc of interstitial packing for a total bed volume of 60 cc. The interstitial packing included α-alumina granules having a particle size in the range from about 28 to 48 mesh. The bed volume corresponded to a reaction zone of about 18 cm length, α-alumina granules having a particle size in the range from about 14 to about 28 mesh was utilized as a preheat inert material and Denstone^{®} 57 tablets ceramic bead support media having a length and width of 3 mm X 3 mm was utilized as a post catalyst inert material. The preheat zone was about 35 cm long and the post catalyst zone was about 30 cm long. The reactor was jacketed to accommodate a recirculating hot oil bath which heated the reactor, and ISCO Model 2350 HPLC pumps were used to pump a feed into the reactor. The feed included a synthetic blend of 95% ethanol and 5% isopropanol.

An on-line Agilent 6890 series gas chromatograph with DB-1701 capillary column having dimensions 60m X 0.32 mm X 1µm, both available from Agilent Technologies of Santa Clara, CA, and an FID detector were utilized to analyze the conversion of the feed. The heated lines to the gas chromatograph were maintained at a temperature in the range from about 130 °C to about 150°C. The conversion, reaction rate and yield results of the feed using Example 1 and Comparative Example 2 are provided in Table 4.

**Table 4: Reaction rate and yield results at constant conversion after 50 hours reaction time on a feed stream at 220 °C, 10 bars absolute, 1 liquid hourly space-velocity.**

| | **% Ethanol conversion** | **Reaction rate, mols ethanol reacted/kg catalyst/h** | **Ethyl acetate yield, g/kg catalyst/h** |
|---|---|---|---|
| **Example 1** | 25.7 | 23.1 | 957 |
| **Comparative Example 2** | 25.5 | 12.6 | 529 |

Reference throughout this specification to "one embodiment," "certain embodiments," "one or more embodiments" or "an embodiment" means that a particular feature, structure, material, or characteristic described in connection with the embodiment is included in at least one embodiment of the invention. Thus, the appearances of the phrases such as "in one or more embodiments," "in certain embodiments," "in one embodiment" or "in an embodiment" in various places throughout this specification are not necessarily referring to the same embodiment of the invention. Furthermore, the particular features, structures, materials, or characteristics may be combined in any suitable manner in one or more embodiments.

## Claims

1. A process of preparing a dehydrogenation catalyst comprising about 10 to about 75 weight % CuO, about 5 to about 50 weight % ZnO, about 1 to about 30 weight % ZrO₂, and about 5 to about 40 weight % alumina, the process comprising the steps:
a) forming a slurry by peptizing alumina having a dispersibility of at least about 50% or greater in an acid at a pH between 2 and 5 and a temperature of about 20°C to 30°C, wherein *"dispersibility"* refers to the percentage of alumina that is less than 1 µm in size after peptizing at a pH from about 2 to about 5,
b) forming a slurry of zirconyl nitrate and water at a pH of less than about 1.5 and a temperature in the range of about 20°C to 30°C,
c) mixing the slurry of a) with the slurry of b) to provide a mixed slurry and maintaining the pH of the mixed slurry at less than about 1.5 and a temperature in the range of about 20°C to 30°C;
d) forming a solution of copper nitrate and zinc nitrate at a pH of less than about 1.5 and at a temperature in the range of about 30°C to 50°C,
e) mixing the solution of d) with the mixed slurry of c) and maintaining the pH at less than about 1.5 and raising the temperature to a range of about 30°C to 50°C to create an acidic slurry containing copper nitrate, zinc nitrate, zirconyl nitrate, and alumina;
f) forming a basic solution of sodium carbonate or sodium bicarbonate at a temperature in the range of about 30°C to 50°C,
g) forming a heel of water in a separate vessel at a temperature in the range of about 30°C to 50°C, and
h) simultaneously adding the acidic slurry (e) and the basic solution of f) to the vessel with the heel of water of h) so that a precipitation reaction occurs at a pH of about 6 to 7 and at a temperature in the range of about 30°C to 50°C to provide a precipitate slurry.

2. The process of claim 1, further comprising:
i) aging the precipitation slurry of h) for a time in the range of about 15 minutes to 15 hours at a temperature in the range of about 30°C to 70°C,
j) filtering and washing the precipitation slurry to provide a filter cake,
k) drying the filter cake; and
1) calcining the dry filter cake to decompose carbonates to oxides.

3. The process of claim 1, wherein the alumina has dispersibility of at least about 70% or greater, preferably at least about 90% or greater.

4. The process of claim 1, wherein the alumina is selected from boehmite, pseudoboehmite, and mixtures thereof.

5. A dehydrogenation catalyst comprising about 10 to about 75 weight % CuO, about 5 to about 50 weight % ZnO, about 1 to about 30 weight % ZrO₂, and about 5 to about 40 weight % alumina, wherein the dehydrogenation catalyst is obtainable by the process according to claim 2.

6. The catalyst of claim 5, wherein the catalyst exhibits an XRD pattern containing boehmitic peaks at 2 theta values of about 14.2° and 28.1°.

7. A method for dehydrogenating an alcohol comprising contacting an alcohol-containing stream with a catalyst of claim 5, which has been reduced in a stream containing hydrogen, and converting the alcohol to an ester.

8. The method of claim 7, wherein the alcohol comprises ethanol and the ester comprises ethyl acetate.

## Patentansprüche

1. Verfahren zum Herstellen eines Dehydrierungskontaktkatalysators, umfassend zu etwa 10 bis etwa 75 Gew.-% CuO, zu etwa 5 bis etwa 50 Gew.-% ZnO, zu etwa 1 bis etwa 30 Gew.-% ZrO₂ und zu etwa 5 bis etwa 40 Gew.-% Aluminium, das Verfahren umfassend die Schritte:
a) Bilden einer Aufschlämmung durch Peptisieren von Aluminium, das eine Dispergierbarkeit von mindestens etwa 50 % oder mehr in einer Säure bei einem pH-Wert zwischen 2 und 5 und einer Temperatur von etwa 20 °C bis 30 °C aufweist, wobei sich *"Dispergierbarkeit"* auf den Prozentsatz von Aluminium bezieht, das eine Größe von weniger als 1 µm nach dem Peptisieren bei einem pH-Wert von etwa 2 bis etwa 5 aufweist,
b) Bilden einer Aufschlämmung aus Zirkonylnitrat und Wasser bei einem pH-Wert von weniger als etwa 1,5 und einer Temperatur in dem Bereich von etwa 20 °C bis 30 °C,
c) Mischen der Aufschlämmung von a) mit der Aufschlämmung von b), um eine gemischte Aufschlämmung bereitzustellen, und Halten des pH-Werts der gemischten Aufschlämmung bei weniger als etwa 1,5 und einer Temperatur in dem Bereich von etwa 20 °C bis 30 °C;
d) Bilden einer Lösung von Kupfernitrat und Zinknitrat bei einem pH-Wert von weniger als etwa 1,5 und bei einer Temperatur in dem Bereich von etwa 30 °C bis 50 °C,
e) Mischen der Lösung von d) mit der gemischten Aufschlämmung von c) und Halten des pH-Werts auf weniger als etwa 1,5 und Erhöhen der Temperatur auf einen Bereich von etwa 30 °C bis 50 °C, um eine saure Aufschlämmung zu erzeugen, die Kupfernitrat, Zinknitrat, Zirkonylnitrat und Aluminium enthält;
f) Bilden einer basischen Lösung von Natriumcarbonat oder Natriumbicarbonat bei einer Temperatur in dem Bereich von etwa 30 °C bis 50 °C,
g) Bilden einer Wasserschicht in einem separaten Behältnis bei einer Temperatur in dem Bereich von etwa 30 °C bis 50 °C, und
h) gleichzeitiges Hinzufügen der sauren Aufschlämmung (e) und der basischen Lösung von f) zu dem Behältnis mit der Wasserschicht von h), so dass eine Fällungsreaktion bei einem pH-Wert von etwa 6 bis 7 und bei einer Temperatur in dem Bereich von etwa 30 °C bis 50 °C stattfindet, um eine Fällungsaufschlämmung bereitzustellen.

2. Verfahren nach Anspruch 1, ferner umfassend:
i) Altern der Fällungsaufschlämmung von h) für eine Zeit in dem Bereich von etwa 15 Minuten bis 15 Stunden bei einer Temperatur in dem Bereich von etwa 30°C bis 70 °C,
j) Filtern und Waschen der Fällungsaufschlämmung, um einen Filterkuchen bereitzustellen,
k) Trocknen des Filterkuchens; und
I) Kalzinieren des trockenen Filterkuchens, um Carbonate zu Oxiden zu zersetzen.

3. Verfahren nach Anspruch 1, wobei das Aluminium eine Dispergierbarkeit von mindestens etwa 70 % oder mehr, vorzugsweise mindestens etwa 90 % oder mehr, aufweist.

4. Verfahren nach Anspruch 1, wobei das Aluminium aus Böhmit, Pseudoböhmit und Mischungen davon ausgewählt ist.

5. Dehydrierungskontaktkatalysator, umfassend zu etwa 10 bis etwa 75 Gew.-% CuO, zu etwa 5 bis etwa 50 Gew.-% ZnO, zu etwa 1 bis etwa 30 Gew.-% ZrO₂ und zu etwa 5 bis etwa 40 Gew.-% Aluminium, wobei der Dehydrierungskontaktkatalysator durch das Verfahren nach Anspruch 2 erhalten werden kann.

6. Kontaktkatalysator nach Anspruch 5, wobei der Kontaktkatalysator ein XRD-Muster zeigt, das böhmitische Spitzen bei 2-Theta-Werten von etwa 14,2° und 28,1° enthält.

7. Verfahren zum Dehydrieren eines Alkohols, umfassend ein Inkontaktbringen eines alkoholhaltigen Stroms mit einem Kontaktkatalysator nach Anspruch 5, der in einem Wasserstoff enthaltenden Strom reduziert wurde, und Umwandeln des Alkohols in einen Ester.

8. Verfahren nach Anspruch 7, wobei der Alkohol Ethanol umfasst und der Ester Ethylacetat umfasst.

## Revendications

1. Procédé de préparation d'un catalyseur de déshydrogénation comprenant environ 10 à environ 75 % en poids de CuO, environ 5 à environ 50 % en poids de ZnO, environ 1 à environ 30 % en poids de ZrO₂ et environ 5 à environ 40 % en poids d'alumine, le procédé comprenant le étapes suivantes :
a) la formation d'une suspension par peptisation d'alumine présentant une dispersibilité d'au moins environ 50 % ou plus dans un acide à un pH compris entre 2 et 5 et une température d'environ 20 °C à 30 °C, la « *dispersibilité* » faisant référence au pourcentage d'alumine dont la taille est inférieure à 1 µm après peptisation à un pH d'environ 2 à environ 5,
b) la formation d'une suspension de nitrate de zirconyle et d'eau à un pH inférieur à environ 1,5 et à une température dans la plage d'environ 20 °C à 30 °C,
c) le mélange de la suspension de a) avec la suspension de b) pour fournir une suspension mixte et le maintien du pH de la suspension mixte à moins d'environ 1,5 et une température dans la plage d'environ 20 °C à 30 °C ;
d) la formation d'une solution de nitrate de cuivre et de nitrate de zinc à un pH inférieur à environ 1,5 et à une température dans la plage d'environ 30 °C à 50 °C,
e) le mélange de la solution de d) avec la suspension mixte de c) et le maintien du pH à moins d'environ 1,5 et l'augmentation de la température à une plage d'environ 30 °C à 50 °C pour créer une suspension acide contenant du nitrate de cuivre, du nitrate de zinc, le nitrate de zirconyle et l'alumine ;
f) la formation d'une solution basique de carbonate de sodium ou de bicarbonate de sodium à une température dans la plage d'environ 30 °C à 50 °C,
g) la formation d'un talon d'eau dans un récipient séparé à une température dans la plage d'environ 30 °C à 50 °C, et
h) l'ajout simultané de la suspension acide (e) et de la solution basique de f) au récipient avec le talon d'eau de h) de sorte qu'une réaction de précipitation se produit à un pH d'environ 6 à 7 et à une température dans la plage d'environ 30 °C à 50 °C pour fournir une suspension de précipité.

2. Procédé selon la revendication 1, comprenant en outre :
i) le vieillissement de la suspension de précipité de h) pendant un temps dans la plage d'environ 15 minutes à 15 heures à une température dans la plage d'environ 30 °C à 70 °C,
j) le filtrage et le lavage de la suspension de précipité pour fournir un gâteau de filtration,
k) le séchage du gâteau de filtration ; et
I) la calcination du gâteau de filtration sec pour décomposer les carbonates en oxydes.

3. Procédé selon la revendication 1, dans lequel l'alumine présente une dispersibilité d'au moins environ 70 % ou plus, de préférence d'au moins environ 90 % ou plus.

4. Procédé selon la revendication 1, dans lequel l'alumine est choisie parmi la boehmite, la pseudoboehmite et leurs mélanges.

5. Catalyseur de déshydrogénation comprenant environ 10 à environ 75 % en poids de CuO, environ 5 à environ 50 % en poids de ZnO, environ 1 à environ 30 % en poids de ZrO₂ et environ 5 à environ 40 % en poids d'alumine, le catalyseur de déshydrogénation pouvant être obtenu par le procédé selon la revendication 2.

6. Catalyseur selon la revendication 5, dans lequel le catalyseur manifeste un motif XRD contenant des pics boehmitiques à 2 valeurs thêta d'environ 14,2° et 28,1 °.

7. Procédé de déshydrogénation d'un alcool comprenant la mise en contact d'un flux contenant de l'alcool avec un catalyseur selon la revendication 5, qui a été réduit dans un flux contenant de l'hydrogène, et la conversion de l'alcool en un ester.

8. Procédé selon la revendication 7, dans lequel l'alcool comprend de l'éthanol et l'ester comprend de l'acétate d'éthyle.
